# EUROPEAN PATENT APPLICATION

(11) **EP 2 689 912 A2**
(43) Date of publication of application: **29.01.2014**
(21) Application number: 13177843.3
(22) Date of filing: 24.07.2013
(51) Int. Cl.: B29C 49/42, A61L 2/12, A61L 2/18, B29C 49/46

(54) **Method and apparatus for forming a container**

(30) Priority: 25.07.2012 IT PR20120048
(71) Applicant: Gea Procomac S.p.A., 43038 Sala Baganza (PR) (IT)
(72) Inventor: Pagliarini, Paolo, 43038 SALA BAGANZA (PR) (IT)
(74) Representative: Gotra, Stefano

(57) **Abstract**

Method for forming a container for food products starting from a preform (10) made of plastic material, said preform (10) comprising an opening (11) and a bottom (12) opposite to the opening (11), the method comprising the steps of:
-introducing into the preform an agent (15) intended to perform a sterilising action, said agent (15) if treated with microwaves being heated up more quickly than the material of the preform (10);
-directing the microwaves towards the preform (10).

The step of introducing said agent (15) takes place before directing the microwaves towards the preform (10) and envisages collecting said agent (15) on the bottom (12) of the preform (10); the step of directing the microwaves towards the preform (10) envisages the evaporation of the agent (15) and the condensation thereof in contact with the preform (10); the step of directing the microwaves towards the preform (10) further envisaging the re-evaporation of the agent (15) condensed on the preform (10) and the subsequent softening of the preform (10) itself for its shaping.

## Description

The present invention relates to a method and an apparatus for forming a container for food products.

An apparatus for forming a container for food products of the type described in US2011/0272861 is known. This apparatus comprises a turntable placed upstream from a stretch blowing station of the preforms. The turntable comprises a plurality of gripping elements each intended to project within a corresponding preform in order to grasp it. The gripping elements each comprise a corresponding dispensing nozzle of a sterilising agent. Furthermore each preform can be moved by the gripping means to be introduced into an annular structure in which it is heated by microwaves.

The sterilising agent is introduced when the heating of the preforms has already begun. This heating takes place by exposing the preforms to microwaves. Hence the sterilising agent comes into contact with the inside walls of the preform which are (already hot) below the gripping means that obstruct the preform in proximity to the opening.

A drawback of this embodiment is connected with the constructive complexity of gripping means that integrate the dispensing nozzle of the sterilising agent within them and with the system needed to dose, vaporise, thermally insulate and distribute the sterilising agent on the rotating manifold. A further drawback of the embodiment indicated above is that neither the inside or the outside portions of the preform near the mouth are subjected to a sterilisation process.

In this context, the technical task underpinning the present invention is to provide a method and an apparatus for forming a container starting from a preform which obviates the drawbacks of the prior art cited above.

In particular, the object of the present invention is to provide a method and an apparatus for sterilising a preform and forming a container that allows improved distribution of the sterilising agent on the preform; a further object is that of simplifying the dosing of the sterilising agent; this also translates into a reduction of the system complexity for the dosing system and the distribution of a sterilising agent (with a substantial reduction in the associated costs and a more extensive sterilising action and lower consumption of the sterilising agent). An important object of the present invention is that of sterilising not only the inside parts of the preform, but also parts of the preform (both inside and outside) located in proximity to the mouth. The technical task set and the objects specified are substantially attained by a method and an apparatus for forming a container, comprising the technical characteristics as set out in one or more of the accompanying claims.

Further characteristics and advantages of the present invention will more fully emerge from the non-limiting description of a preferred but not exclusive embodiment of an apparatus for forming a container, as illustrated in the accompanying drawings, in which:
- Figure 1 is a schematic plan view of an apparatus according to the present invention;
- Figures 2a-2g show a succession of steps of a method according to the present invention;
- Figures 3 and 4 show a perspective view and a sectional view respectively of a component of the apparatus according to the present invention;
- Figures 5 and 6 show a perspective view and a sectional view respectively of the component of Figures 3 and 4 in a different configuration.

In the appended figures, reference number 1 is used to indicate an apparatus for forming a container for food products starting from a preform made of plastic material.

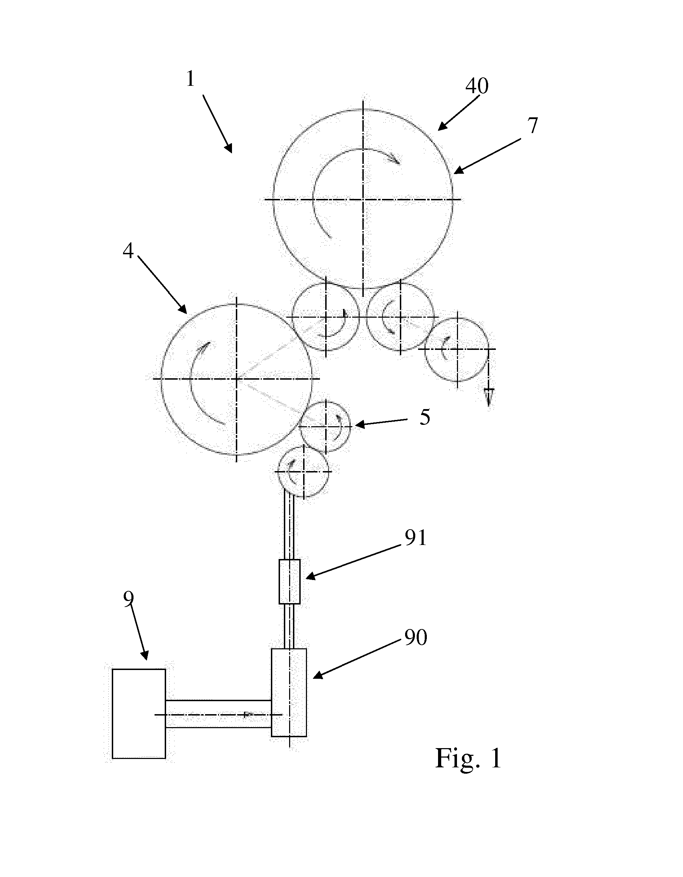

The apparatus 1 advantageously comprises a tank 9 from which the preforms are picked up (see Figure 1). Downstream there are alignment means 90 of the preforms in one or more rows. Subsequently there are dust removal means 91 of the preforms. The tank, the alignment means 90 and the dust removal means 91 are of the known type and therefore not described further.

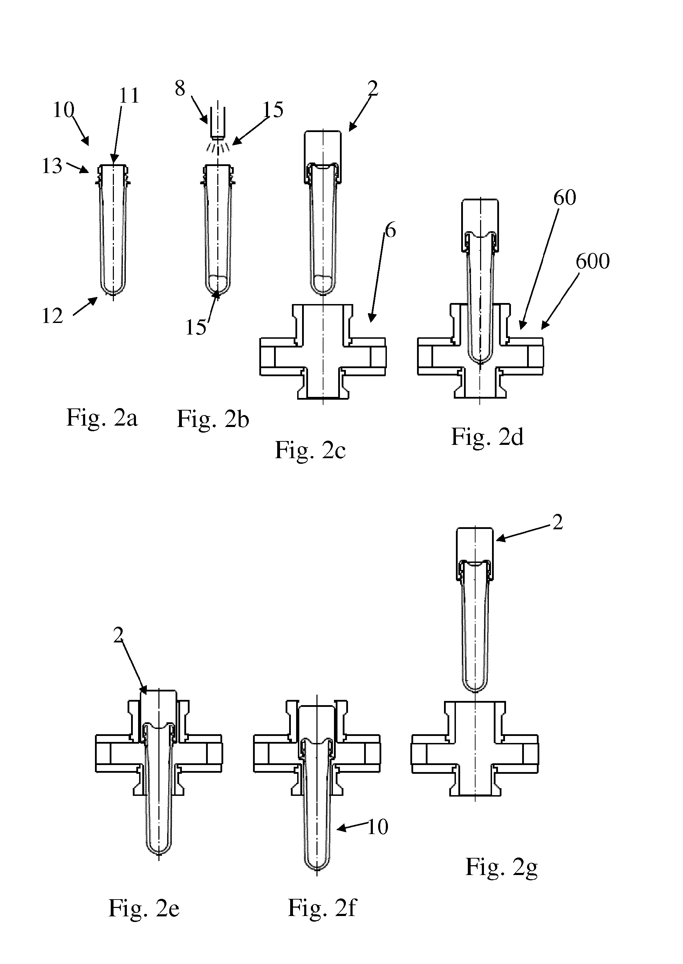

The apparatus 1 (see Figure 2b) comprises introduction means 5 into the preform 10 of an agent 15 intended to perform a sterilising action. Advantageously the sterilising agent 15 can be thermally activated. At high temperatures it has a greater sterilising action. This agent is introduced in the liquid state (even if during the introduction the sterilising agent could be partially nebulised into tiny particles of liquid). Appropriately the introduction means 5 are placed downstream of the alignment means 90. These introduction means 5 advantageously comprise a dosing nozzle 8 of the known type. For example this dosing nozzle 8 may be of the type used for dosing/introducing liquid nitrogen into jars. Advantageously the agent 15 is more sensitive to the heating generated by microwaves with respect to the material of the preform 10. For example, the agent 15 comprises hydrogen peroxide. In an alternative embodiment the agent 15 is water. In that case the sterilising action is connected with the fact that at high temperatures water (potentially in the form of vapour) also performs a sterilising action.

Appropriately a movement line of the preforms develops in correspondence with the nozzle 8, but the nozzle 8 remains in a predetermined position along the movement line. In other words, at least in the preferred embodiment the nozzle 8 does not move integrally with the preforms 10.

The apparatus 1 further comprises microwave directing means 6 towards the preform 10 (see Figures 2c-2g). On this point the directing means 6 comprise a microwave applicator 600 (which as an example is shaped like an annular element inside which the preform can transit). With reference to Figure 1, appropriately the directing means 6 rest on a rotating turntable 4, below also referred to as a rotating heating turntable. In the preferred embodiment the rotating turntable 4 comprises a plurality of heating stations each comprising corresponding microwave directing means 6. These heating stations are distributed along the edge of the turntable 4. With respect to a movement path of the preform along the apparatus 1, the introduction means 5 into the preform of the agent 15 are placed upstream from the microwave directing means 6 towards the preform. The rotating heating turntable 4 also comprises gripping means 2 of the preform 10 which, in combination with the preform itself, identify an annular cavity 3 which externally surrounds the neck 13 of the preform 10 and which is in fluid communication with the inside of the preform 10 (see Figures 2c, 4 and 6). Each heating station is associated with corresponding gripping means 2. Appropriately it is the movement of the gripping means 2 that allows the preforms to move towards and away from the corresponding microwave directing means 6. Appropriately the gripping means 2 comprise a gripper 20 better described below.

The apparatus 1 further comprises a forming station 7 (typically a blowing or stretch-blowing one). The forming station 7 is placed downstream of the microwave directing means 6 (see Figure 1). Preferably the station 7 rests on a dedicated turntable 40.

The present invention further relates to a method for forming a container for food products starting from a preform 10 made of plastic material. Appropriately this method is implemented by an apparatus 1 having one or more of the technical characteristics described above.

The preform 10 comprises an opening 11 and a bottom 12 opposite to the opening 11. The opening 11 is placed in correspondence with a neck 13 of the preform.

Appropriately the method envisages the following preliminary steps (please refer to Figure 1):
- taking a succession of preforms 10 from a tank 9;
- putting the preforms 10 in order according to a movement row;
- performing a dust removal procedure.

The method comprises the steps of introducing into the preform 10 an agent 15 intended to perform a sterilising action (see Figure 2b). This advantageously takes place after performing the preliminary steps indicated above. The step of introducing the agent 15 envisages dispensing the agent 15 through a nozzle 8 (advantageously of the type described above). During said step of introducing the agent 15 the nozzle 8 remains still while the preforms 10 move below it.

The agent 15 is introduced into the preform 10 in liquid form. The agent 15, if treated with microwaves, heats up more quickly than the material of the preform 10. Typically the preforms are usually made of PET. The agent 15 advantageously comprises/is hydrogen peroxide. The latter is approximately 100-1000 times more sensitive to microwaves than PET. As indicated above, the agent 15 could be water.

The method therefore envisages the step of directing the microwaves towards the preform 10. In this way the fast movement of the dipoles of the sterilising agent 15 induced by the microwaves determines much quicker heating of the sterilising agent 15 with respect to the PET.

The step of directing the microwaves towards the preform 10 takes place advantageously in a heating turntable 4.

The step of introducing said sterilising agent 15 takes place before directing the microwaves towards the preform 10 and envisages collecting said sterilising agent 15 on the bottom 12 of the preform 10 (see Figure 2c); Just before the start of the step of directing the microwaves towards the preform 10, the sterilising agent 15 is completely or almost completely on the bottom 12 of the preform 10.

The step of introducing the sterilising agent 15 into the preform 10 takes place upstream from the heating rotating turntable 4.

The step of directing the microwaves towards the preform 10 envisages the preform being introduced into the microwave applicator 600.

The step of directing the microwaves towards the preform 10 therefore envisages (causes) the evaporation of the sterilising agent 15 (compare Figures 2c and 2d). The step of directing the microwaves towards the preform 10 first envisages the exposure to the microwaves only of the bottom of the preform 10 and of the sterilising agent 15 collected on the bottom 12. Only subsequently will the exposure to the microwaves of the remaining parts of the preform take place. The evaporation of the sterilising agent 15 by the microwaves is instantaneous (it usually takes less than 1 second, even if this depends on the powers at stake and the mass of the sterilising agent 15). Because of the reasons indicated above, when the sterilising agent 15 evaporates the preform 10 will be colder than the sterilising agent 15. Consequently the sterilising agent 15 condenses in contact with the preform 10. The evaporation step of the sterilising agent 15 collected in the preform 10 therefore causes part of the sterilising agent 15 to exit through the opening 11 in the preform 10 and the condensation of the sterilising agent 15 onto an outside portion of the preform 10. The evaporation step upwards of the sterilising agent from the bottom 12 of the preform 10 promotes the optimal distribution thereof along all the inside walls of the preform and thanks to the annular cavity 3 also in correspondence with the outside surface of the neck 13.

As indicated above, the step of introducing the preform into the applicator 600 takes place by inserting the bottom 12 of the preform first (see Figure 2d). In this way the sterilising agent 15 located there evaporates before the substantial heating of other parts of the preform 10 (so the evaporated sterilising agent finds the preform still at a low temperature which facilitates the subsequent condensation). Subsequently portions of the preform 10 gradually closer to the opening 11 are progressively introduced into the applicator 600.

The condensation of the sterilising agent 15 on the preform 10 affects the entire inside surface 101 of the preform 10 and part of the outside surface 102 of the preform 10. The sterilising agent 15 that condenses on the preform is more concentrated (in particular the percentage of the sterilising component, for example, hydrogen peroxide is higher than on the bottom 12 of the preform before evaporation). After the condensation of the sterilising agent 15 on the outside of the neck the gripping means 2 and the neck 13 of the preform are introduced into the annular applicator 600 to allow the activation of the sterilising agent condensed there (see Figure 2f).

The step of directing microwaves towards the preform 10 further envisages a re-evaporation of the sterilising agent 15 condensed on the preform 10 (following the previous evaporation). In the event that the sterilising agent 15 comprises hydrogen peroxide the re-evaporation of the agent causes the activation of the agent 15 and appropriately its distribution. Appropriately the step of directing microwaves towards the preform 10 envisages the softening of the preform 10 itself for its shaping (there is a significant softening of the preform only following the re-evaporation of the sterilising agent 15). In this way the energy of the microwaves is used both for the re-evaporation of the sterilising agent 15, and for the heating of the preform 10 according to a predetermined temperature profile.

The step of directing the microwaves towards the preform 10 is preceded by a step of gripping the preform 10 by gripping means 2 acting on the outside of the preform 10 (see Figure 2c). The gripping means 2 identify, in combination with the preform 10 itself, an annular cavity 3. The annular cavity 3 externally surrounds the neck 13 of the preform and is in fluid communication with the inside of the preform 10 to allow the evaporated sterilising agent 15 to reach the outside of the neck 13.

Appropriately at least a part of the evaporated sterilising agent 15 re-condenses on the outside of the neck 13 of the preform 10.

Usually the outside of the neck 13 comprises a threaded part 17 intended for connection with an external cap and preferably the evaporated sterilising agent 15 is distributed across the whole of said threaded part 17 by re-condensing.

The gripping means 2 comprise a gripper 20 which is engaged below a first protrusion 18 of the preform 10 which develops along an annular line surrounding the preform 10. In the technical field the first protrusion 18 is also known as a seal tearing ring. Preferably the whole weight of the preform 10 is unloaded onto said gripper 20.

Appropriately the gripper 20 engages in a groove 93 interposed between said first protrusion 18 and a second protrusion 94. This second protrusion 94 also develops along an annular line that surrounds the preform 10. In the technical field the second protrusion 94 is also known as a neck rim. The gripping step ensures that the preform hangs from the gripping means 2.

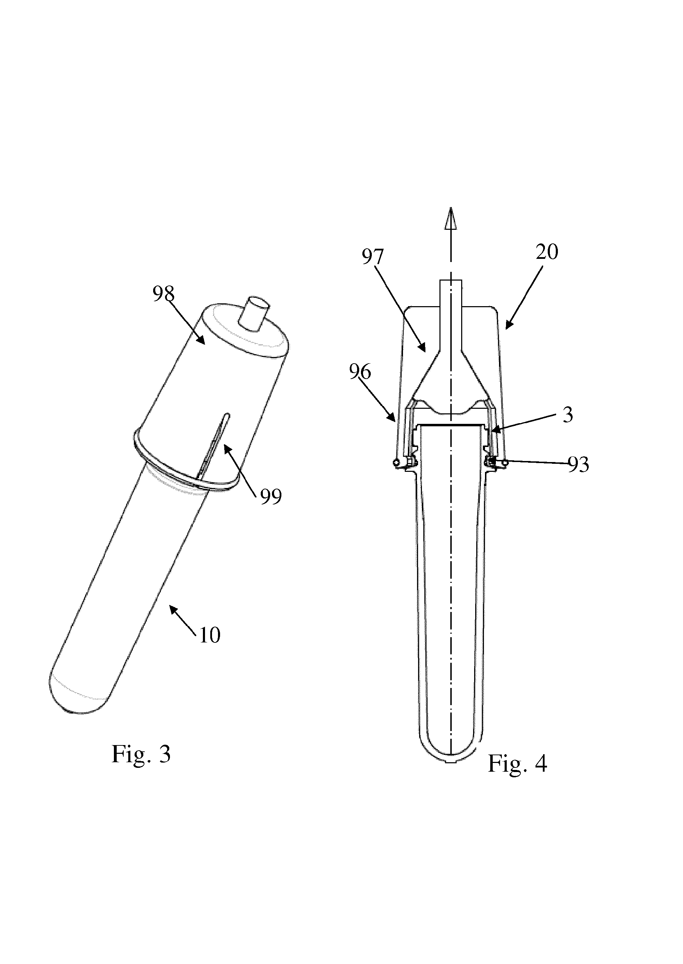

In the preferred embodiment the gripper 20 is made of PTFE. The opening and closing of the gripper 20 may be of various kinds. One of these is shown in Figures 3-6 and reference is made to this by way of example below. Advantageously the gripper 20 comprises a shell 95 which is open at one end (typically the lower end) for the insertion of the preform 10. Appropriately the shell 95 is equipped with a plurality of jaws 96 intended to surround at least a part of the preform 10. The jaws 96 project in a cantilever fashion from a common support 98 being part of the shell 95. The shell 95 has one or more weakening grooves 99 that advantageously separate the jaws 96 from each other.

The gripping step envisages a divarication of the gripping means 2 (typically a divarication of the shell 95), see Figure 4.

The gripping step further comprises the introduction of the neck 13 of the preform inside a shell 95 defined by the gripping means 2.

In particular the divarication step envisages an elastic deformation of the jaws 96 of the gripper 20.

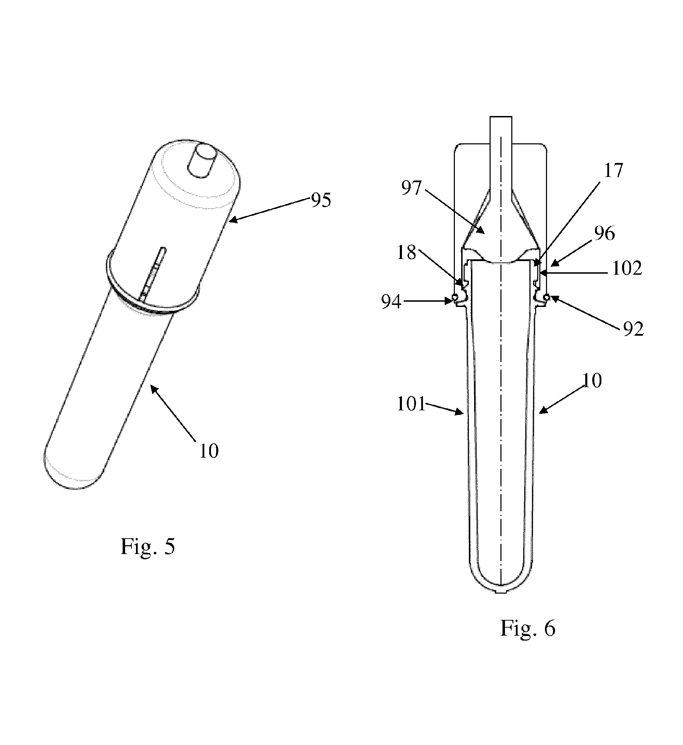

The gripping step further comprises a tightening step on the preform 10 of the gripping means 2 (see Figure 6), such tightening action comprising an elastic return of the gripping means 2. Advantageously the gripper 20 comprises an elastic ring 92 which is in contact with and surrounds the shell 95 (in particular the jaws 96). The divarication step of the shell 95 envisages a deformation of the ring 92 which during the tightening step returns potential elastic energy stored in the divarication step (this detail allows greater tightening force of the preform 10).

During the tightening step the jaws 96 are inserted into said groove 93. To allow the relative shift of the two jaws 96 the gripper 20 can comprise a divaricator 97 sliding along a first direction; this sliding divaricator 97 is mobile between a first position in which it determines the reciprocal movement of the jaws 96 away from each other and a second position in which it enables the jaws 96 to move towards each other. With respect to the shell 95 the divaricator 97 in the first position is higher than in the second position. The divaricator 97 can therefore move integrally with the shell 95 in order to allow a shift of the gripper 20, but can also move in relation to the shell 95 to allow the divarication and tightening of the jaws 96. Appropriately the divaricator 97 comprises a conical surface which can be operatively coupled with a conical surface of the shell 95. The conical surface of the shell 95 has a higher angle of convergence than the angle of convergence of the conical surface of the shell 95. In order to perform the divarication step of the shell 95 the divaricator 97 is raised with respect to the shell 95 and increases the contact area between the conical surface of the divaricator 97 and the conical surface of the shell 95.

In particular the gripping step envisages the gripper 20 surrounding the threaded part 17 of the preform 10. In a particular embodiment (see Figure 2c) the gripping step envisages a part of the gripper 20 projecting into the preform 10 (without coming into contact with the preform 10 and however without completely obstructing the opening 11). Appropriately the part of the gripper 20 that projects into the preform 10 is tapered in order to facilitate the passage of the vaporised sterilising agent 15 from the inside of the preform 10 to the annular cavity 3. After the softening of the preform 10, which takes place in the heating turntable 4, the perform 10 is subjected to a deformation step for the forming of the final container. The forming step mainly takes place by blowing and/or stretch blowing. Typically the forming step takes place in a forming turntable 40 separate from the heating turntable 4.

The invention as it is conceived enables multiple advantages to be attained.

First of all, it allows optimal distribution of the sterilising agent thanks to the evaporation action from the lower bottom of the preform. This optimal distribution is also enabled due to the fact that the preform is externally gripped and therefore there are no obstacles to the distribution of the sterilising agent within the preform, but neither are there any obstacles that prevent the exit of the sterilising agent in the annular cavity 3.

The invention as conceived is susceptible to numerous modifications and variants, all falling within the scope of the inventive concept characterised thereby. Furthermore all the details can be replaced by other technically equivalent element. In practice, all the materials used, as well as the dimensions, can be any according to requirements.

## Claims

1. A method for forming a container for food products starting from a preform (10) made of plastic material, said preform (10) comprising an opening (11) and a bottom (12) opposite to the opening (11), the method comprising the steps of:
- introducing into the preform (10) an agent (15) intended to perform a sterilising action;
- directing the microwaves towards the preform (10);
**characterised in that** the step of introducing said agent (15) takes place before directing the microwaves towards the preform (10) and includes collecting said agent (15) on the bottom (12) of the preform (10);
the step of directing the microwaves towards the preform (10) comprises the evaporation of the agent (15) collected on the bottom (12) of the preform (10) and condensation thereof in contact with the preform (10); the step of directing microwaves towards the preform (10) further comprises the re-evaporation of the agent (15) condensed on the preform (10) and the softening of the preform (10) itself for its shaping.

2. The method according to claim 1, **characterised in that** the step of introducing the agent (15) into the preform (10) envisages introducing said agent (15) in liquid form; at the beginning of the step of directing the microwaves towards the preform (10) the agent (15) being completely or almost completely on the bottom (12) of the preform (10); the step of directing the microwaves towards the preform (10) envisages at first the exposure to the microwaves only of the bottom of the preform (10) and of the agent (15) collected on the bottom and subsequently the exposure to the microwaves of remaining parts of the preform (10); the exposure to the microwaves of the bottom (12) of the preform (10) being at least in part accompanied by the evaporation of the agent (15).

3. The method according to claim 1 or 2, **characterised in that** the evaporation step of the agent (15) collected in the preform (10) is followed and/or accompanied by the exit of part of the agent (15) through the opening (11) of the preform (10) and the condensation of the agent (15) on an outside portion of the preform (10).

4. The method according to any of the previous claims, **characterised in that** the evaporation step of the agent (15) by means of the microwaves is instantaneous; said agent (15) if treated with microwaves being heated up more quickly than the material of the preform (10).

5. The method according to any of the previous claims, **characterised in that** the step of directing the microwaves towards the preform (10) is preceded by pick-up step of the preform (10) using gripping means (2) acting on the outside of the preform (10); said gripping means (2) identifying, in combination with the preform (10) itself, an annular cavity (3); said annular cavity (3) externally surrounding the neck (13) of the preform and being in fluid communication with the inside of the preform (10) so as to allow the evaporated agent (15) to reach the outside of the neck (13).

6. The method according to any of the previous claims, **characterised in that** the step of introducing the agent (15) takes place upstream from a rotating heating turntable (4) in which the step of directing the microwaves towards the preform (10) takes place; the step of introducing the agent (15) envisages delivering the agent (15) through a nozzle (8); during said step of introducing the agent (15) the nozzle (8) remains still whereas the preforms (10) move below;
after the softening of the preform (10), which takes place in the heating turntable (4), the preform (10) is subjected to a forming process of the final container, said forming process taking place in a forming turntable (40) separate from the heating turntable (4).

7. The method according to any of the previous claims, **characterised in that** the condensation of the agent (15) on the preform (10) affects the entire inside surface (101) of the preform (10) and part of the outside surface (102) of the preform (10).

8. The method according to any of the previous claims, **characterised in that** the softening of the preform (10) is subsequent to the re-evaporation of the agent (15).

9. An apparatus for forming a container for food products starting from a preform made of plastic material, said apparatus comprising:
- introduction means (5) into the preform of a liquid agent destined to have a sterilising effect, said agent being more sensitive to the heat generated by microwaves with respect to the material of the preform;
- directing means (6) of microwaves towards the preform;
- a forming station (7) placed downstream from the microwave directing means (6);
**characterised in that**, with respect to a movement path of the preform, said introduction means (5) into the preform of said agent are placed upstream from the microwave directing means (6) towards the preform.

10. The apparatus according to claim 9, **characterised in that** the microwave directing means (6) towards the preform are afforded on a rotating turntable which also comprises gripping means (2) of the preform (10); the gripping means (2) of the preform identifying, in combination with the preform itself, an annular cavity (3); said annular cavity externally surrounding the neck (13) of the preform (10) and being in fluid communication with the inside of the preform (10).
